(19) Europäisches Patentamt
European Patent Office
Office européen des brevets

(11) Publication number: 0 141 662 A2

(12) # EUROPEAN PATENT APPLICATION

(21) Application number: 84307592.0

(22) Date of filing: 02.11.84

(51) Int. Cl.[4]: C 01 B 25/36, C 01 B 33/28, B 01 J 29/04

(30) Priority: 04.11.83 NZ 206157
11.11.83 NZ 206232

(43) Date of publication of application: 15.05.85
Bulletin 85/20

(84) Designated Contracting States: BE DE FR GB IT NL

(71) Applicant: NEW ZEALAND GOVERNMENT PROPERTY CORPORATION, Parliament Buildings, Wellington (NZ)

(72) Inventor: Wright, Leonard James, 4 Penguin Drive, Murray's Bay Auckland (NZ)
Inventor: Milestone, Neil Brennan, 5 Bedford Grove, Lower Hutt (NZ)

(74) Representative: Collier, Jeremy Austin Grey et al, J.A.Kemp & Co. 14, South Square Gray's Inn, London WC1R 5EU (GB)

(54) **Crystalline substituted aluminophosphates and their preparation and use.**

(57) The invention provides novel microporous crystalline aluminophosphates in which some of the aluminium ions have been substituted by certain divalent atoms, and some of the phosphate ions can be substituted by certain tetravalent atoms. As a result, acid sites are formed and the crystal lattice sets upper and lower limits to the size of molecules that can be operated on and produced. In particular, the substituted aluminophosphate crystals are catalysts useful for converting methanol into useful hydrocarbons.

EP 0 141 662 A2

# CRYSTALLINE SUBSTITUTED ALUMINOPHOSPHATES AND THEIR PREPARATION AND USE

This invention relates to crystalline, substituted aluminophosphates and to methods of preparing them.

It is known, for example in European Patent EP0043562, that a series of crystalline microporous aluminophosphates can be prepared where the chemical composition expressed in terms of molar ratios of oxides, is claimed to be

$$Al_2O_3 : 1 \pm 0.2\ P_2O_5$$

and the pore size is in the range 3A to 10A. These microporous aluminophosphates can be prepared by hydrothermal treatment of aluminophosphate gels containing a templating or structure-direction agent.

The crystal frameworks of these aluminophosphates are neutral and inactive, and they exhibit little or no catalytic activity. In particular, they cannot be used to produce hydrocarbons from methanol. They are useful for sieving molecules according only to their dimensions. For this reason, they have been suggested as suitable as inert supports for materials such as Group VIII metals which can then act as catalysts.

More recently European patent application 105512 and American patent 4440871 disclose the formation of silicon substituted aluminophosphates by the hydrothermal treatment of an active form of silica together with aluminium hydroxide and phosphoric acid in the presence of a templating species, usually an organic base. These materials were shown in patent application EP 105512 to be catalytic and to convert methanol to non-petroleum hydrocarbons, including light olefins.

The present invention provides a broader range of compositions of microporous aluminophosphates than has

hitherto been available. This objective is achieved by modifying the structure of the crystals by substituting foreign ions into some of the lattice sites usually occupied by aluminium or phosphorus and so producing new materials which act as catalysts without impregnation or loading.

The novel substituted microporous crystalline aluminophosphates of the present invention have a pore diameter of 3 to 10 A, the substitution in which causes the formation of acid sites when calcined, and of the formula:

$$Al_2O_3 : 1 \pm 0.4\ P_2O_5 : x\ MO_n$$

where $0 < x < 0.4$, M is a metal with a valency of 2 or 4 or a mixture of such metals, n is 1 when M is divalent and n is 2 when M is tetravalent. The invention includes such catalysts both in the uncalcined (precursor) and calcined states.

Preferred such catalysts are those of the formula:

$$Al_2O_3 : 1.2 \pm 0.2\ P_2O_5 : x\ MO$$

where x is as hereinbefore defined and M is magnesium, zinc, manganese, cobalt, nickel, or iron; and those of formula:

$$Al_2O_3 : 0.8 \pm 0.2\ P_2O_5 : x\ MO_2$$

where x is as hereinbefore defined and M is any one or more of germanium (IV), tin (IV), titanium (IV), silicon (IV), germanium (II), tin (II), or titanium (II).

The invention provides a process for preparing a substituted microporous crystalline aluminophosphate which comprises

(1) forming a reaction mixture with a composition expressed in mole ratios of the oxides of:

$$Al_2O_3 : 1 \pm 0.5\ P_2O_5 : MO_n : 20\text{-}80\ H_2O$$

together with 0.5-2.5 moles of at least one templating species; and

(2) heating this mixture at a temperature of at least $90_oC$ under autogenous conditions until crystals of substituted

0141662

aluminophosphate are formed.

The invention consists of the foregoing, but by way of example only, some substituted aluminophosphates will be discussed with reference where relevant to the accompanying Figures, in which:

<u>Figure 1</u> shows the infra-red spectrum of pyridine adsorbed on an unsubstituted aluminophosphate in the region of 1 800 - 1 300 $cm^{-1}$, after the $AlPO_4$ bands have been subtracted.

<u>Figure 2</u> shows the infra-red spectrum of pyridine adsorbed on a Si substituted aluminophosphate in the region of 1 800-1 300 $cm^{-1}$.

<u>Figure 3</u> a thermogravimetric trace of weight loss in an unsubstituted aluminophosphate precursor.

<u>Figure 4</u> a thermogravimetric trace of weight loss of a Ge substituted aluminophosphate precursor.

<u>Figure 5</u> a trace showing release of chemical species with temperature as determined by mass spectrometry for a Ge substituted aluminophosphate precursor.

In Figure 1 very few Bronsted acid sites are shown by the unsubstituted aluminophosphates as evidenced by the small intensity of the protonated pyridine adsorption band at 1543 $cm^{-1}$.

In Figure 2 an enhanced number of Bronsted acid sites are shown by Si-substituted aluminophosphates.

In Figure 3 the thermogravimetric trace of an unsubstituted aluminophosphate shows no high temperature weight loss.

In Figure 4, the derivative weight loss (DTG) clearly shows the two steps of the weight loss, one centred at 220°C due to physisorbed amine while the peak at 480°C shows loss due to the protonated species associated with the acid sites.

Figure 5 shows the generation of templating amine, alkene and ammonia as lost from a Ge-substituted aluminophosphate. The weight loss at 210°C is associated with the templating molecule (plus a small amount of water) and at 480°C the loss of alkene and ammonia caused by Hoffman degradation of a protonated amine.

It has already been stated that microporous alumino-phosphates will sieve a mixture of molecules of suitable size, but will not catalyse reactions. We wished to develop

methods of, among other things, producing useful hydrocarbons from methanol. This reaction can be catalysed in a number of ways, using strong acids (e.g. Chem. Comm. 1974 p.397 and Chem. Letters 1979 p.849). Such acid treatment is not selective, so that the products are a wide mixture of hydrocarbons, not suitable for any particular use.

Examples 14 and 15 which follow, give a good indication of the use of the acid sites in substituted aluminophosphates as catalysts for producing hydrocarbons from methanol where the products required can be selected by choosing the pore diameter of the catalyst.

When substituted, aluminophosphates have the potential to produce ion exchangeable materials, and some such materials are catalytic. While the ion exchange reaction itself does not constitute catalysis, it is not possible to have catalytic activity without ion exchange. If aluminophosphates are to be used as acid catalysts, then substitution must occur so that a hydrogen form can be obtained; a sodium form will not act as a catalytic material. It was important to find which elements could be substituted into the crystal lattice while still retaining the microporous crystalline structure.

Prior work lists Si as the only known atom which substitutes into the aluminophosphate lattice. This invention has found that a further 9 elements can be usefully substituted for some of the aluminium or phosphorus in the lattice and so produce acid sites and hence a structure which may act as a catalyst. The introduction of divalent ions into a lattice made up of tetrahedral units of $AlO_2$ and $PO_2$ is novel and not previously investigated with respect to the formation of acid sites. The substitution of B or Ga for Al or As or V for P would produce materials which do not necessarily have acid sites which produce catalytic activity.

A tetravalent atom which has been substituted for phosphorus which has a valency of 5, creates a crystal lattice in which there is a negative charge associated with

the substitution site. In a similar way, a divalent ion can be substituted for aluminium which has a valency of 3 to produce the same type of site. An exchangeable cation will be associated with the negatively charged site caused by the substituted atom. An exchangeable proton is necessary for the initiation of a reaction which involves protonation as an important part of the reaction mechanism of an organic reaction such as in the formation of hydrocarbons from methanol.

The present substituted aluminophosphate compositions exhibit novel surface selectivity characteristics which also render them useful as catalyst bases in a number of hydrocarbon conversion and oxidative combustion reactions. They can be impregnated or otherwise loaded with catalytically active metals by methods well known in the art and used, for example, in fabricating catalysts compositions having silica or alumina bases.

The as-synthesised substituted aluminophosphate, herein termed the precursor, has a composition which has a general formula

$$Al_2O_3 : 1.2 \pm 0.2\ P_2O_5 : x\ MO_2 : n\ H_2O\ m\ R$$

where $0 < x < 0.4$

and where M may be any one or more of Mg
Zn
Mn
Co
Ni
Fe

or it has the general formula

$$Al_2O_3 : 0.8 \pm 0.2\ P_2O_5\ y\ MO_2\ n\ H_2O\ m\ R$$

where $o < y < 0.4$

and where M may be one of Ge, Sn, Ti or any two or more of Si, Ge, Sn, Ti

and $7 < n < 100$ and $0.1 < m < 3$

or where the divalent or tetravalent species can coexist.

Water and the templating agent R do not form an integral part of the crystal lattice, but reside within the pores and channels of the lattice. The templating agent can usually be removed by calcination in air or oxygen. Some of this species can be removed by mild processes such as gentle heating or evacuation.

It has been found preferable to incorporate the substituting elements into the crystal lattice as it is formed by adding them in a suitable reactive chemical form to the reaction mixture before crystallization has occurred. They are incorporated in the crystal lattice during crystallization. The balancing elements can be added to the reaction mixture before crystallization in the same way, or, after crystallization, by such a process as ion exchange. In certain cases, the templating organic base can act as a charge balancing species.

It has been found convenient to prepare the crystals which are the subject of this application by hydrothermal crystallization of a reaction mixture prepared by combining a reactive source of phosphate, alumina, water, at least one structure-directing or templating agent (which is often an organic amine or quaternary ammonium salt), a chemically reactive source of the substituting element or elements, and a chemically reactive source of charge balancing element or elements. The latter do not need to be specially added, but can be counter-ions for the balancing ions, and include phosphate or aluminium ions.

It has been found convenient to heat the reaction mixture under autogenous pressure in a sealed, inert, pressure vessel at an elevated temperature which can be between 90°C and 300°C and is preferably between 150°C and 250°C until crystallization has occurred, which may take between a few hours and a few weeks.

Some preferred general methods of preparing the present class of substituted crystalline microporous aluminophosphates and the results of applying these methods are illustrated in the following examples.

Example 1

Phosphoric acid (88%) (4.74 ml) hydrated aluminium oxide (66.41% $Al_2O_3$), (5.430g), water (20.7 ml), triethylamine (7.5 ml) and cobaltous chloride hexahydrate (0.256g) were mixed until homogeneous and heated at 200°C under autogenous pressure in an inert vessel for 24 hours. The resulting product was cooled, the solid filtered and washed well with water and dried at ambient temperature. The resulting blue crystalline material gave an x-ray powder diffraction pattern* with the following major peaks for values of 2θ between 4° and 40°.

Table 1

| 2θ | d | I |
|---|---|---|
| 7.7 | 11.48 | VVS |
| 13.2 | 6.71 | W |
| 15.2 | 5.83 | M |
| 20.1 | 4.42 | VS |
| 21.2 | 4.21 | VS |
| 22.7 | 3.93 | S |
| 24.9 | 3.58 | W |
| 26.4 | 3.38 | S |
| 29.2 | 3.06 | W |
| 30.5 | 2.93 | M |
| 33.9 | 2.64 | W |
| 35.0 | 2.56 | M |
| 37.7 | 2.39 | W |

(errors in values of 2θ estimated to be ± 0.5°C throughout)

*Cu K radiation used.

This pattern of peaks is similar to that described for the aluminophosphate designated $AlPO_4$-5, in European Patent EP0043562.

The material was heated in air at 200°C for two hours, then at 500°C for 24 hours. The material turned pale yellow in colour and gave an x-ray powder diffraction pattern with the following major peaks:

TABLE 2

| 2θ | d | I |
|---|---|---|
| 7.4 | 11.95 | VS |
| 12.9 | 6.86 | M |
| 15.0 | 5.91 | W |
| 19.8 | 4.84 | S |
| 21.1 | 4.21 | VS |
| 22.4 | 3.97 | VVS |
| 26.0 | 3.43 | S |
| 29.2 | 3.06 | M |
| 30.2 | 2.96 | M |
| 33.8 | 2.65 | W |
| 34.6 | 2.59 | M |
| 37.1 | 2.42 | W |
| 37.9 | 2.37 | W |

EXAMPLES 2 - 6

A procedure similar to that in example 1 was followed except where indicated in table 3 below:

TABLE 3

| Example | Amount of hydrated aluminium oxide used (g) | Amount of $CoCl_2.6H_2O$ used (g) |
|---|---|---|
| 2 | 5.499 | 0.0426 |
| 3 | 4.572 | 0.1281 |
| 4 | 5.348 | 0.5124 |
| 5 | 5.182 | 1.0248 |
| 6 | 4.962 | 1.7079 |

The crystalline solids produced in examples 2 - 6 were all blue and displayed x-ray powder diffraction patterns similar to that described in table 1. The intensity of the blue colouration appeared to increase as the amount of $CoCl_2.6H_2O$ added to the initial reaction mixture increased.

On calcining in air the materials all turned yellow and gave x-ray powder diffraction patterns similar to that described in table 2.

## EXAMPLE 7

A procedure similar to that in example 1 was followed except that the $CoCl_2.6H_2O$ was substituted by $ZnSO_4.7H_2O$ (0.3096g).

## EXAMPLE 8

The same procedure as example 1 was followed except 0.39g ground $GeO_2$ was added.

Both these examples gave white crystalline materials with x-ray powder diffraction patterns very similar to that described in table 1. After calcining in air, the materials gave x-ray powder diffraction patterns similar to that described in table 2.

## EXAMPLE 9

Phosphoric acid (88%) (6.57 ml), hydrated aluminium oxide (68.84% $Al_2O_3$ by weight) (7.41g), water (29.75 ml), di-n-propylamine (6.83 ml) and cobalt chloride hexahydrate (0.3769g) were mixed until homogeneous and heated at 200°C under autogenous pressure in an inert pressure vessel for 24 hours. The resulting product was cooled the solid filtered and washed well with water and dried at ambient temperature. The resulting blue crystalline material gave an x-ray powder diffraction pattern with the following major peaks for values of 2θ between 4° and 40°.

TABLE 4

| 2θ | d | I |
|---|---|---|
| 8.2 | 10.78 | W |
| 9.6 | 9.21 | M |
| 13.3 | 6.66 | W |
| 15.8 | 5.61 | M |
| 20.5 | 4.33 | S |
| 21.2 | 4.19 | VVS |
| 22.3 | 3.99 | S |
| 22.8 | 3.90 | VS |
| 23.3 | 3.82 | VS |
| 24.9 | 3.58 | W |
| 26.8 | 3.33 | W |
| 28.8 | 3.10 | W |
| 29.6 | 3.02 | W |
| 31.7 | 2.82 | W |
| 33.1 | 2.71 | W |
| 34.4 | 2.61 | W |
| 38.0 | 2.37 | W |

This pattern of peaks is similar to that described for the aluminophosphate designated $AlPO_4$-11 in European patent EP0043562.

The material was heated in air at 200°C for 2 hours then at 500° for 24 hours. The yellow/green product then gave an x-ray powder diffraction pattern similar to that described in table 5 below. This is similar to that described for the calcined $AlPO_4$-11 described in European patent EP0043562.

TABLE 5

| 2θ | d | I |
|---|---|---|
| 8.2 | 10.8 | W |
| 8.5 | 10.4 | M |
| 9.8 | 9.0 | M |
| 12.8 | 6.9 | M |
| 16.1 | 5.5 | S |
| 19.9 | 4.5 | M |
| 20.3 | 4.4 | M |
| 21.9 | 4.1 | VVS |
| 22.2 | 4.0 | S |
| 22.6 | 3.9 | VS |
| 23.6 | 3.8 | S |
| 24.1 | 3.7 | M |
| 25.8 | 3.5 | M |
| 26.8 | 3.3 | W |
| 27.3 | 3.3 | W |
| 27.8 | 3.2 | M |
| 29.8 | 3.0 | S |
| 30.4 | 2.9 | M |
| 31.8 | 2.8 | M |
| 32.7 | 2.7 | M |
| 38.8 | 2.3 | W |

EXAMPLE 10

A procedure similar to that described in example 9 was followed except that instead of adding cobalt chloride hexahydrate, stannic oxide (0.68 g) was used. The resulting white product gave an x-ray powder diffraction pattern similar to that described in Table 4, and after calcining in air it gave an x-ray powder diffraction pattern similar to that described in Table 5.

In the examples cited above, as well as Si substituted aluminophosphates prepared in a similar way to those claimed in US Patent 4,440,871 substitution occurs so that acid sites are formed within the crystal lattice. A recognised technique for

investigating the characteristics of acidic sites in zeolites is pyridine adsorption studied by infra red spectroscopy. (see Ward. "Zeolite Chemistry and Catalysis" A.C.S. Monograph 171 Ed. J.R. Rabo). Adsorption of pyridine onto Bronsted acid sites gives rise to a characteristic band at 1545 $cm^{-1}$, while adsorption on Lewis sites gives rise to a band at 1450 $cm^{-1}$.

## EXAMPLE 11

The Si-substituted aluminophosphates as claimed in US Patent 4440871 provides a useful tool for demonstrating the presence of acid sites in substituted aluminophosphates by studying pyridine adsorption by infrared spectroscopy. A Si-substituted aluminophosphate was pressed into a self-supporting disc and supported in a cell in the beam of a Fourier transform infra-red spectrometer. It was dried in a stream of nitrogen at 300°C. The pyridine was introduced as a vapour at 200°C and the absorbance spectrum was recorded. The spectrum of the chemisorbed pyridine, after subtraction of the $AlPO_4$ bands, is given in Figures 1 and 2, for both substituted and unsubstituted aluminophosphates, showing that a significant increase in the number of Bronsted acid sites occurs upon substitution into the crystal lattice.

Thermogravitry/Mass Spectrometry was used by Parker, Bibby and Patterson to investigate the acid sites formed in ZSM-5 precursors (Zeolites 4 168-178, 1984). A high temperature (350°C-500°C) weight loss was shown to indicate the presence of protonated amines held at the acid site of ZSM-5, caused by aluminium substituted into the silicate lattice. An aluminophosphate prepared according to examples given in European Patent EP0043562 gives no such weight loss. (Fig. 3).

## EXAMPLE 12

In a series of substituted aluminophosphates prepared according to examples 1 - 10, a high temperature weight loss is shown by thermogravimetry (Fig. 4). Mass spectrometry shows that this loss is caused by loss of alkene and ammonia caused by Hoffman degradation of the protonated amine at the acid site. In heating example 8 where

triethylamine is the templating ion, the loss is due to ethylene and ammonia, (Fig. 5), while for example 10 the weight loss is due to propene and ammonia caused by decomposition of the dipropylamine chemically bound at the acid site. The presence of ion exchange is another accepted method for indicating the presence of charged sites within a zeolite framework.

EXAMPLE 13

A calcined sample prepared according to example 6 was exchanged with ammonium nitrate followed by washing and calcination, to ensure that the substituted aluminophosphate was a hydrogen form. Treatment of this H-form with sodium hydroxide resulted in the release of hydrogen ions. Measurement of the resultant pH change indicated the release of hydrogen ions corresponding to 2 mole % substitution of the aluminophosphate.

It is considered that the present invention will be found most useful in catalysing a number of hydrocarbon conversion reactions as well as the formation of hydrocarbons from oxygenates in particular methanol.

The crystalline substituted microporous aluminophosphates described above have been found to catalyse at elevated temperatures the conversion of methanol to a mixture of hydrocarbon products. Under certain conditions the major products formed include various hydrocarbons and water. The range of products produced is determined by the pore size of the aluminophosphate structure. Preferably the reaction temperature used is in the region 150°C to 500°C.

EXAMPLE 14

As an example of a typical conversion procedure a sample of calcined substituted crystalline microporous aluminophosphate (1g), prepared as described in example 8 was placed in a sealed vertical glass reactor tube containing a sintered glass frit at the bottom. The tube was electrically heated to a temperature of 360°C and methanol (approx. 1 ml

per hour) slowly introduced at a constant rate by means of a mechanical pump onto a glass wool pad at the top of the reactor. The methanol vapour thus formed then slowly percolated through the catalyst bed where conversion took place. The reaction products passed out of the bottom of the reactor tube through the sintered glass frit and were collected and analysed.

Essentially complete conversion of methanol to hydrocarbon products and water was observed. Gaseous, liquid and solid hydrocarbons were formed. Some of the hydrocarbon products identified include methane, ethane, ethene, propane, propene, n-butane, iso-butane, butenes, higher alkanes and alkenes, benzene, and various methylated benzenes. In particular, significant quantities of pentamethyl- and hexamethylbenzene were formed. A typical product distribution is given in Table 6.

EXAMPLE 15

A sample of the calcined substituted, crystalline microporous aluminophosphate prepared as in example 9 was treated with methanol as described in example 14 above. Again essentially complete conversion of methanol to hydrocarbons and water was observed. In this case only gaseous hydrocarbons and a mixture of liquid hydrocarbons were formed.

Some of the components of the gaseous phase included methane, ethane, ethene, propane, propene, n-butane, butenes and _iso_-butane. Some of the components of the liquid phase included higher alkanes benzene and various methylated benzenes. The proportions of pentamethyl- and hexamethylbenzene formed under these conditions were considerably less than those formed in example 14. The major products are given in Table 6.

TABLE 6

| | Example 14 | Example 15 |
|---|---|---|
| | Weight % of hydrocarbon products | |
| Gases ($C_1$ - $C_4$) | 35-45% | 50-40% |
| Liquids | 50-25% | 45-55% |
| Solids (hexamethylbenzene) | 15-30% | <5% |

Composition of liquid product (wt %)

| Component | Example 14 | Example 15 |
|---|---|---|
| Alkanes $C_5$ - $C_9$ | 63.8 | 75.8 |
| Benzene | 0.4 | 0.4 |
| Toluene | 1.6 | 0.7 |
| Ethylbenzene | 0.3 | 1.0 |
| p-Xylene | 0.4 | 0.4 |
| m-Xylene | 0.9 | 0.8 |
| o-Xylene | 1.1 | 1.1 |
| p-Ethyltoluene | 0.1 | 0.3 |
| m-Ethyltoluene | 0.3 | 0.3 |
| o-Ethyltoluene | 0.2 | 0.3 |
| 1,3,5-Trimethylbenzene | 0.4 | 0.4 |
| 1,2,4-Trimethylbenzene | 2.7 | 0.9 |
| 1,2,3-Trimethylbenzene | 0.6 | 0.8 |
| Diethyl- and Ethyldimethylbenzenes | 0.7 | 1.0 |
| Durene and Isodurene | 8.0 | 1.1 |
| 1,2,3,4-Tetramethylbenzene | 1.6 | 0.1 |
| Pentamethylbenzene | 13.7 | 3.7 |
| Hexamethylbenzene | 0.3 | 3.1 |
| Unidentified higher aromatics (including napthalenes) | 2.9 | 7.8 |

## CLAIMS

1. A substituted microporous crystalline aluminophosphate having a pore diameter from 3 to 10 A; and the formula:

$$Al_2O_3 : 1 \pm 0.4\ P_2O_5 : X\ MO_n$$

where $0 < x < 0.4$, M is a metal with a valency of 2 or 4 or a mixture of such metals, n = 1 when M is divalent, and n = 2 when M is tetravalent; the substitution being such that acid sites are formed, in the precalcined and calcined state.

2. A substituted microporous crystalline aluminophosphate as claimed in claim 1 of the formula

$$A\ l_2O_3 : 1.2 \pm 0.2\ P_2O_5 : x\ MO$$

where x is as defined in claim 1 and M is any one or more of magnesium, zinc, manganese , cobalt, nickel and iron.

3. A substituted microporous crystalline aluminophosphate as claimed in claim 1 of the formula

$$Al_2O_3 : 0.8 \pm 0.2 : x\ MO_2$$

where x is as defined in claim 1 and M is any one or more of germanium (IV), tin (IV), titanium (IV), silicon (IV), germanium (II), tin (II) or titanium (II).

4. A substituted microporous crystalline aluminophosphate as claimed in claim 1 wherein the acid sites formed on calcination are such as to catalyse the transformation of methanol to hydrocarbons and such that the composition of the hydrocarbons formed is a function of the pore size of the said substituted aluminophosphate.

5. A substituted microporous crystalline aluminophosphate as claimed in claim 4 wherein the pore size is between 5 and 10 A and such that the percentages of hydrocarbon products formed from methanol are greater than 3% pentamethyl benzene, and 15% hexamethyl benzene.

6. A substituted microporous crystalline aluminophosphate as claimed in claim 4 wherein the pore size lies between 3 and 8 A, and the liquid hydrocarbons formed from methanol are suitable for gasoline.

. 7. A substituted microporous crystalline aluminophosphate as claimed in claim 4 such as to catalyse methanol conversion between 150°C and 500°C.

8. A process for preparing a substituted microporous crystalline aluminophosphate as claimed in claim 1 which comprises:

(1) forming a reaction mixture with a composition, expressed in mole ratios of the oxides, of

$$Al_2O_3 : 1 \pm 0.5\ P_2O_5 : x\ MO_n : 20\text{-}80\ H_2O$$

where M and x are as defined in claim 1, together with 0.5 - 2.5 moles of at least one templating species; and

(2) heating this mixture at a temperature of at least 90°C under autogenous pressure until crystals of the substituted aluminophosphate are formed.

9. A process according to claim 8 wherein the reaction mixture is heated to a temperature between 90°C and 120°C for a period which depends on the elements in the reaction mixture, and is then heated at 150-250°C for up to two weeks.

10. A process for producing hydrocarbons which comprises contacting methanol at elevated temperature with a substituted microporous crystalline aluminophosphate as claimed in any of claims 1 to 7 which has been calcined.

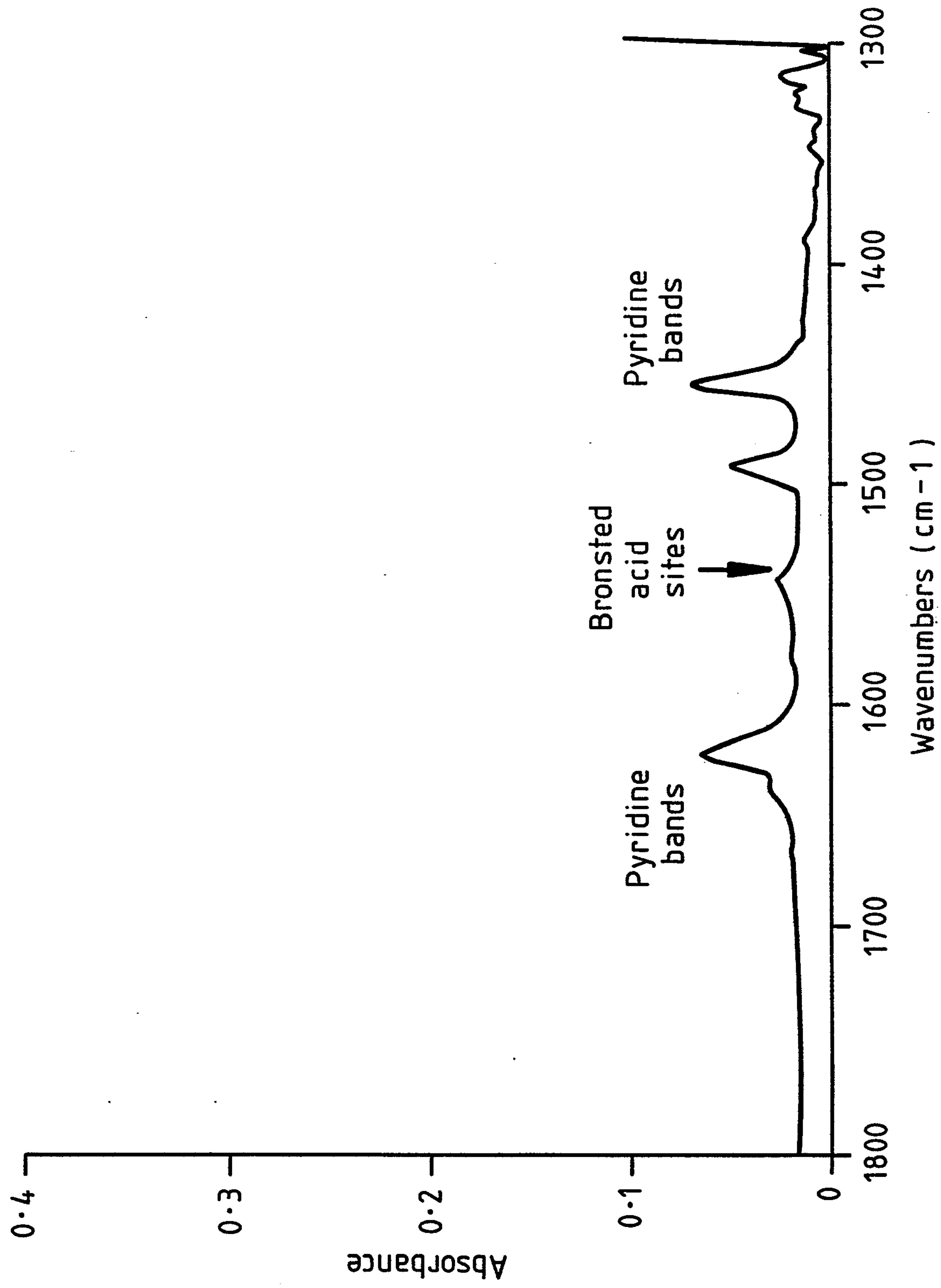


Unsubstituted ALPO4−11
Figure 1

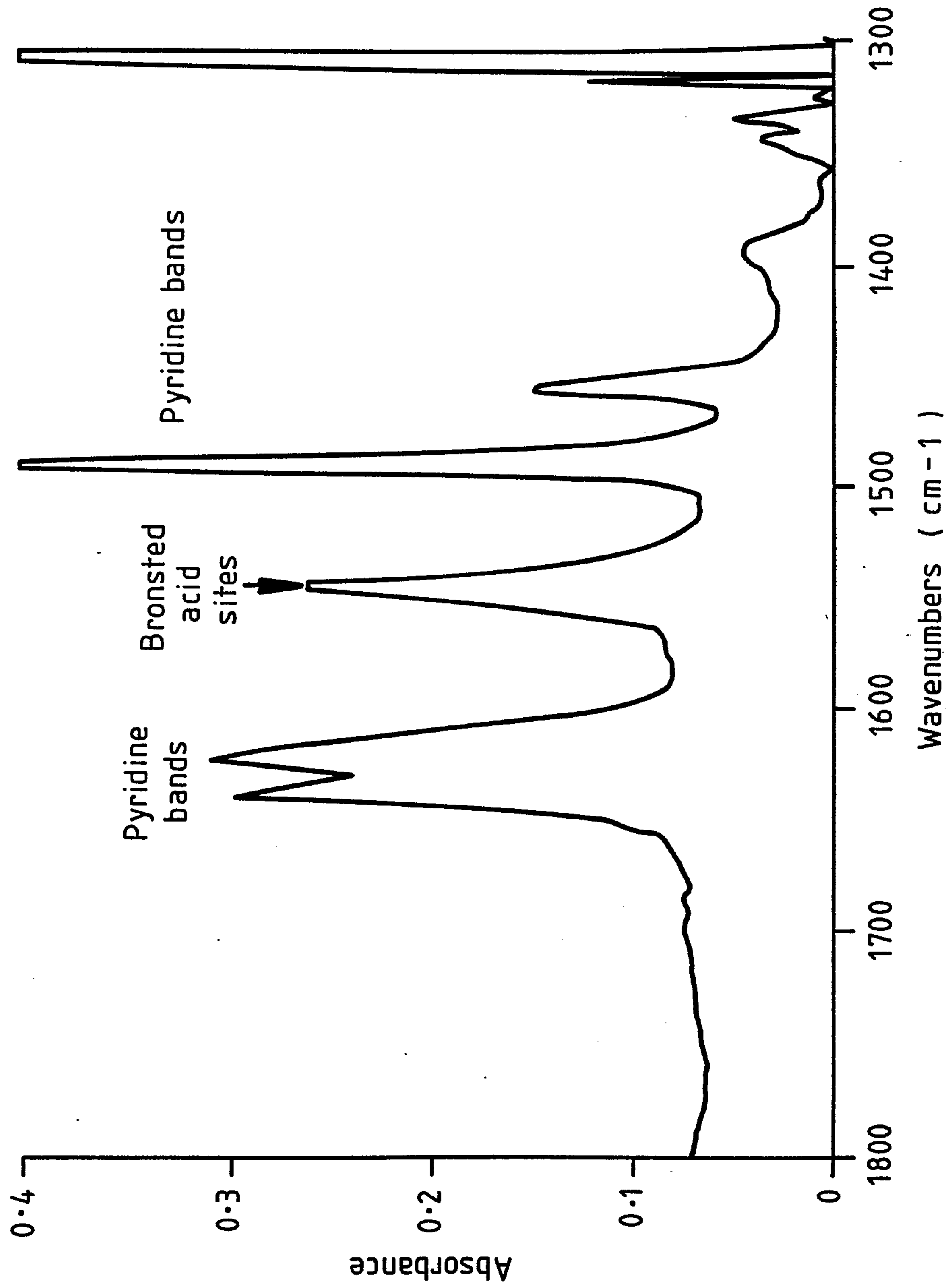


Substituted ALPO4-11

Figure 2

0141662

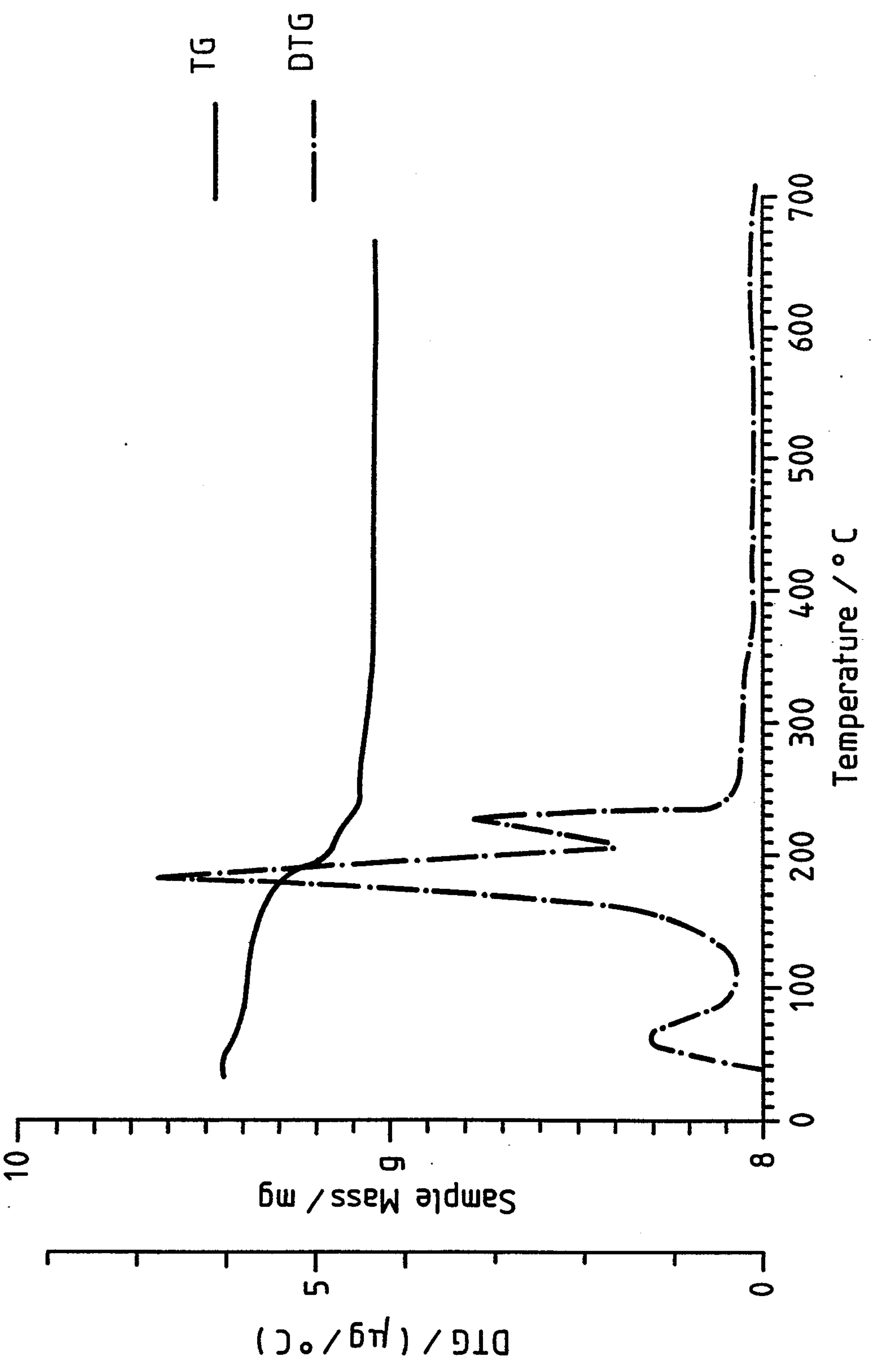


Unsubstituted ALPO4-11 Precursor
Figure 3

0141662

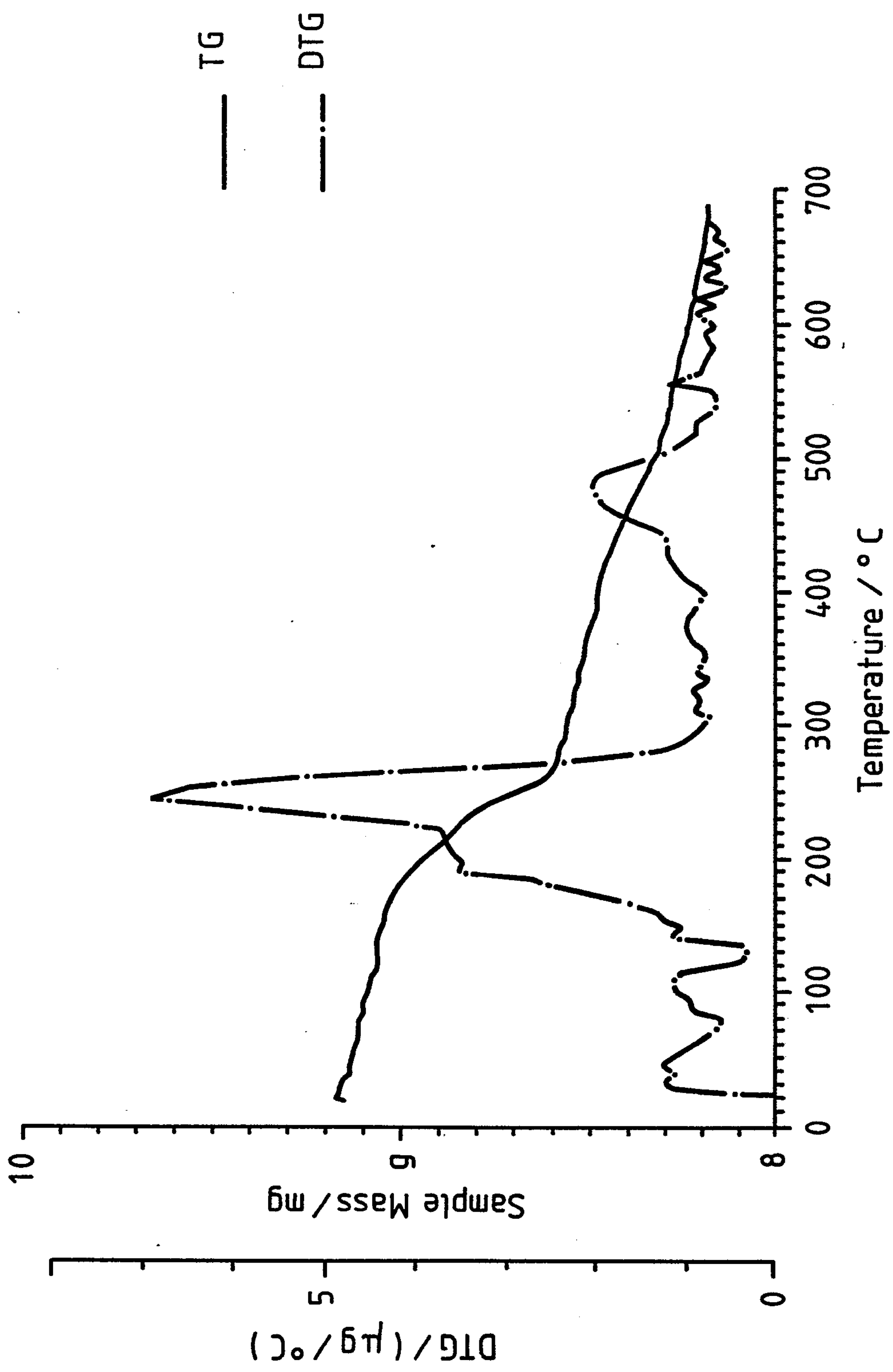


Substituted ALPO4-11 Precursor

Figure 4

0141662

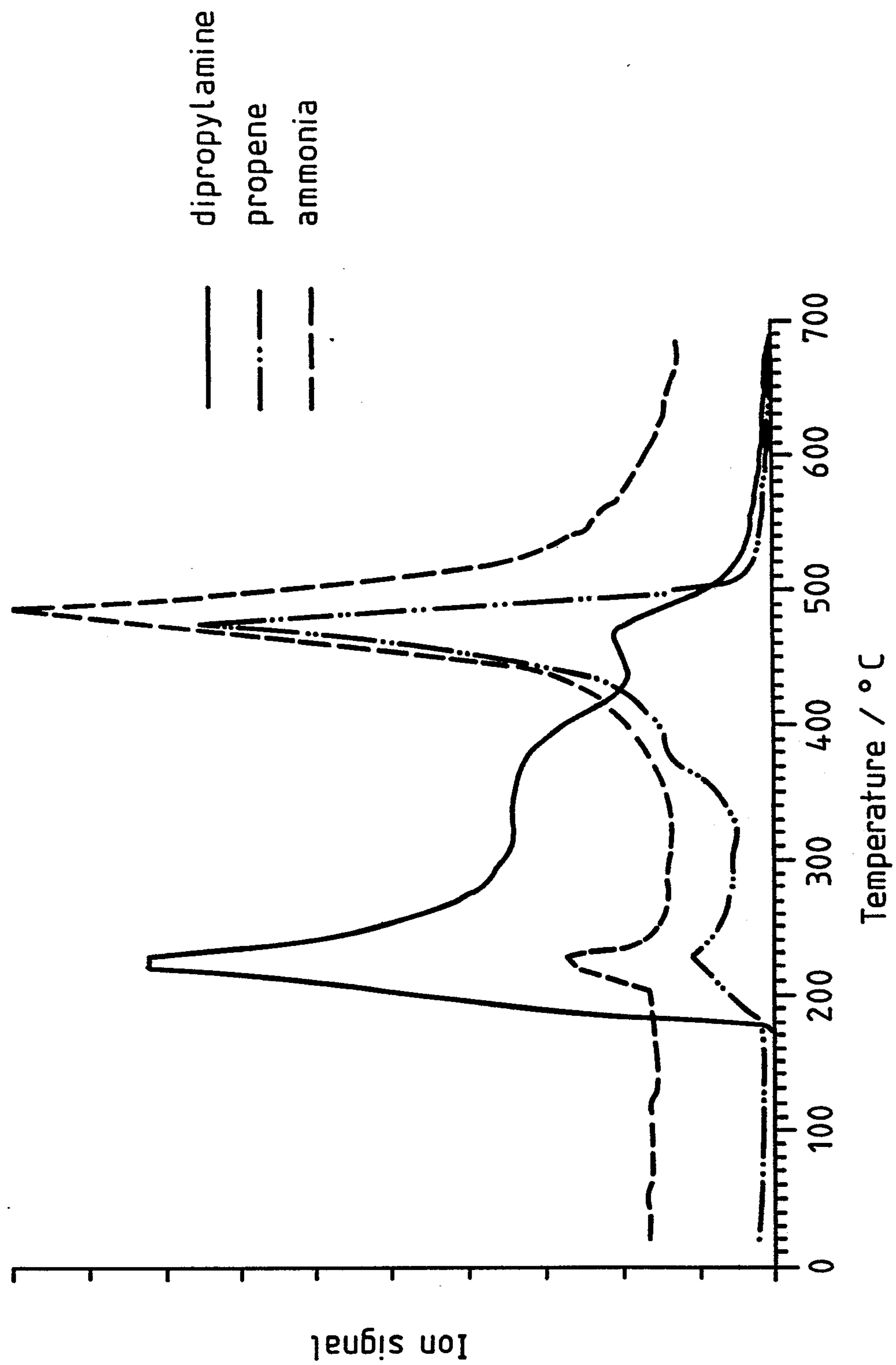


Substituted ALPO4-11 Precursor

Figure 5